# EUROPÄISCHE PATENTSCHRIFT

(11) **EP 4 021 347 B1**
(45) Veröffentlichungstag und Bekanntmachung des Hinweises auf die Patenterteilung: **21.05.2025**
(21) Anmeldenummer: 20753741.6
(22) Anmeldetag: 07.08.2020
(51) Int. Cl.: A61F 2/16

(54) **INTRAOKULARLINSE MIT EINEM LÄNGENVERÄNDERLICHEN BÜGEL ALS HAPTIK**
INTRAOCULAR LENS COMPRISING AN ARM WITH AN ADJUSTABLE LENGTH AS A HAPTIC
LENTILLE INTRAOCULAIRE COMPRENANT UN BRAS AYANT UNE LONGUEUR RÉGLABLE EN TANT QU'ÉLÉMENT HAPTIQUE

(30) Priorität: 30.08.2019 DE 102019123300
(43) Veröffentlichungstag der Anmeldung: 06.07.2022
(73) Patentinhaber: Carl Zeiss Meditec AG, 07745 Jena (DE)
(72) Erfinder: MÜLLER, Stephanie, 16816 Neuruppin (DE)
(74) Vertreter: Hofstetter, Schurack & Partner
(86) Internationale Anmeldenummer: PCT/EP2020/072217
(87) Internationale Veröffentlichungsnummer: WO 2021/037524

(56) Entgegenhaltungen:
- WO-A1-2014/058316
- GB-A- 2 518 378
- US-A- 4 134 161
- US-A1- 2004 111 152
- US-A1- 2011 313 523
- US-A1- 2017 312 071

## Beschreibung

### Technisches Gebiet

Ein Aspekt der Erfindung betrifft eine Intraokularlinse mit einem optischen Teil und mit einer Haptik, die mit dem optischen Teil gekoppelt ist, und mit einer optischen Hauptachse, die eine Vorderseite und eine Rückseite des optischen Teils durchdringt, wobei die Haptik ein erstes Haptikteil aufweist, welches als strangartiger Bügel ausgebildet ist, wobei der Bügel eine Längsachse aufweist.

### Stand der Technik

Intraokularlinsen sind in vielfältigen Ausgestaltungen bekannt. Üblicherweise weisen Intraokularlinsen zumindest zwei separate Haptiken auf, die in Umlaufrichtung um die optische Hauptachse gegenüberliegend ausgebildet sind und radial anschließend an den optischen Teil ausgebildet sind. Es können auch mehr als zwei derartige separate Haptiken ausgebildet sein, beispielsweise drei Haptiken.

Intraokularlinsen können an unterschiedlichen definierten Positionen im Auge anstelle einer natürlichen Linse des Auges implantiert werden. So ist es in dem Zusammenhang vorgesehen, dass spezifische Intraokularlinsen in einer Vorderkammer des Auges implantiert werden. Beispielsweise können derartige Vorderkammerlinsen im vorderen Kammerwinkel fixiert werden.

Darüber hinaus sind Intraokularlinsen bekannt, die als Iris-Clip-Linsen bezeichnet werden. Derartige Intraokularlinsen werden an der Pupille befestigt. Insbesondere werden sie dabei an die Pupillenöffnung geklemmt. Eine derartige Intraokularlinse ist beispielsweise aus der DE 10 2007 057 122 A1 bekannt. Die dortige Intraokularlinse mit diesem spezifischen Implantationsort im Auge weist zwei gegenüberliegende Haptiken auf. Jede dieser Haptiken weist zwei L-förmig geformte Haptikarme auf. Einander zugewandte Enden dieser Haptikarme sind, in einer Ebene senkrecht zur optischen Hauptachse dieser Intraokularlinse betrachtet, einander zugewandt angeordnet, jedoch berührungslos und überlappungsfrei angeordnet. Mittels derartig gebildeter Haptikarme kann durch den gebildeten Spalt, der in Umlaufrichtung um die optische Hauptachse zwischen den Enden der Haptikarme gebildet ist, die Iris eingeklemmt werden. Derartige Linsen sind jedoch nicht dazu vorgesehen und tauglich, in einem Kapselsack eines Auges implantiert werden zu können.

Diesbezüglich sind weitere spezifische Intraokularlinsen bekannt, die als Hinterkammerlinsen bezeichnet werden können und in einen Kapselsack des Auges implantiert werden.

Aus der US 2004111152 A1 sind alle essentiellen Merkmale der Präambel aus Anspruch 1 bekannt.

Aus der DE 103 10 961 B4 ist eine Intraokularlinse bekannt, die eine Hinterkammerlinse ist. Bei dieser Hinterkammerlinse ist vorgesehen, dass zwei separate Haptiken an gegenüberliegenden Bereichen des optischen Teils radial anschließend an den optischen Teil ausgebildet sind. Beide jeweiligen Haptiken sind mit zwei haptischen Teilen ausgebildet. Die beiden haptischen Teile einer Haptik sind relativ zueinander bewegbar. Dazu ist an einer definierten Verbindungsstelle zwischen den einstückig miteinander verbundenen Haptikteilen eine definierte Knickstelle, beispielsweise als Filmscharnier, ausgebildet. Dadurch kann das radial äußere haptische Teil dieser Haptik gegenüber dem ersten haptischen Teil, welches direkt an dem optischen Teil anschließt, geknickt beziehungsweise verklappt werden. Diese Klappbewegung ist nur in der Ebene senkrecht zur optischen Achse möglich. Dadurch soll dort die radiale Weite der gesamten Intraokularlinse reduziert werden, um Reizungen im Inneren des Kapselsacks durch diese Haptiken vermeiden zu können.

Aus der US 4 077 071 B ist eine Intraokularlinse bekannt, die einfache Bügel als Haptiken aufweist. Diese längensteifen Bügel sind hohl und als Schlauch ausgebildet. Dadurch tritt auch hier das Problem auf, dass sie in unterschiedlich großen Kapselsäcken nur bedingt positionsstabil angeordnet werden können. Ein Kippen und/oder Rotieren der Intraokularlinse im Kapselsack kann auftreten. Dies führt zu Nachteilen beim Sehen.

Aus der US 2011/313523 A1 ist eine Intraokularlinse mit Bügeln bekannt, deren Längen variable sind. Aus der GB 2 518 378 A, der US 4 134 161 A, der WO 2014 058316 A1 und Der US 2017/312071 A1 sind Intraokularlinsen mit Bügeln bekannt.

### Darstellung der Erfindung

Es ist Aufgabe der vorliegenden Erfindung, eine Intraokularlinse zu schaffen, die eine verbesserte Positionierung in einem Kapselsack eines Auges ermöglicht.

Diese Aufgabe wird durch eine künstliche Intraokularlinse gemäß den Merkmalen des Anspruchs 1 gelöst.

Ein Aspekt der Erfindung betrifft eine künstliche Intraokularlinse mit einem optischen Teil. Der optische Teil ist eine Linse. Der optische Teil weist spezifische optische Abbildungseigenschaften auf, sodass damit spezifische Korrekturen von Sehfehlern durchgeführt werden können.

Die Intraokularlinse weist darüber hinaus eine Haptik auf, die mit dem optischen Teil gekoppelt ist. Die Intraokularlinse weist eine optische Achse beziehungsweise eine optische Hauptachse auf, die eine Vorderseite und eine Rückseite des optischen Teils durchdringt und insbesondere zentral mittig durchdringt.

Die Haptik weist zumindest ein erstes Haptikteil auf. Das erste Haptikteil ist als strangartiger Bügel ausgebildet. Dieser Bügel weist eine Längsachse auf. Der Bügel weist zumindest eine Längenveränderungsvorrichtung auf, mit welcher der Bügel selbst in Richtung seiner Längsachse in seiner Länge definiert veränderlich ist. Durch diese Ausgestaltung ist also ein Bügel geschaffen, der in sich selbst in seiner Länge definiert veränderlich ist. Diese Länge ist auch noch in eine bestimmte Richtung, nämlich in Richtung seiner Längsachse betrachtet, veränderlich. Durch eine derartige Ausgestaltung kann die Länge eines derartigen strangartigen Bügels individuell eingestellt werden. Dadurch ergeben sich situationsabhängig definierte unterschiedliche Längen eines Bügels, die somit zu unterschiedlich großen Intraokularlinsen führen. Durch eine derartige variable Längeneinstellung eines spezifischen Teils der Intraokularlinse, nämlich dieses strangartigen Bügels, kann die positionsstabile Implantierung der Intraokularlinse in unterschiedlich große Kapselsäcke erreicht werden. Es müssen somit nicht mehr vielfältige unterschiedliche separate Intraokularlinsen für unterschiedliche Kapselsäcke bereitgestellt werden. Vielmehr ist es nunmehr möglich, mit einem einzigen Typ einer Intraokularlinse durch eine entsprechende Längenanpassung der Haptik eine angepasste Implantationsgröße zu erzeugen, die an eine individuelle Größe eines Kapselsacks angepasst werden kann. Dadurch ist in verbesserter Weise sowohl ein unerwünschtes Verkippen als auch ein unerwünschtes Rotieren der Intraokularlinse in einem Kapselsack verhindert.

Es ist vorgesehen, dass der strangartige Bügel auf einer dem optischen Teil zugewandten Seite eine Öffnung aufweist. In diese Öffnung greift ein Rand des optischen Teils ein sodass der optische Teil in dem Haptikteil gehalten ist. Eine derartige Ausgestaltung, bei welcher die Teilkomponenten der Intraokularlinse separate Teile sind, ermöglichen eine einfache mechanische Verbindung der Teilkomponenten, die dennoch eine hohe und dauerhafte stabile Halterung ermöglicht.

Die Öffnung ist insbesondere als Schlitz ausgebildet. Die Öffnung ist insbesondere in Richtung der Längsachse des Bügels verlaufend.

In einer vorteilhaften Ausführung ist vorgesehen, dass die Längenveränderungsvorrichtung zumindest eine Teleskopverbindung aufweist. Unter einer Teleskopverbindung wird eine Ausgestaltung verstanden, bei welcher zwei separate Teilelemente des Bügels ineinander geführt sind und sich in Richtung der Längsachse des Bügels relativ zueinander bewegen können, insbesondere verschieben können. Dadurch können im gekoppelten und ineinander geführten Zustand der beiden Teilelemente unterschiedliche definierte Längen des Bügels eingestellt werden. Insbesondere ist eine derartige Längenveränderungsvorrichtung als eine Teleskopverbindung ausgebildet. Es kann auch vorgesehen sein, dass die Längenveränderungsvorrichtung zumindest zwei separate Teleskopverbindungen aufweist, die in dem Bügel ausgebildet sind.

Eine Teleskopverbindung ist eine mechanisch stabile Ausgestaltung, die die mechanische Belastbarkeit der Haptik als solche aufrecht erhält. Andererseits ist durch dieses Ineinandergeführtsein von zwei separaten Teilelementen auch eine sehr gerichtete Längenveränderung ermöglicht. Ein unerwünschtes Verkippen der Teilelemente zueinander bei der Längenveränderung ist dadurch verhindert. Nicht zuletzt ist durch eine derartige Teleskopverbindung auch eine gewünschte definierte Längeneinstellung stabil gehalten. Gerade im Überlappungsbereich der ineinander geführten Teilelemente ist durch eine Teleskopverbindung auch eine mechanisch robuste und belastbare Schnittstelle gebildet.

Vorzugsweise ist vorgesehen, dass die Längenveränderungsvorrichtung zumindest ein ziehharmonikaartiges Faltteil aufweist. Dieses ziehharmonikaartige Faltteil ist in Richtung der Längsachse des Bügels elastisch in der Länge veränderbar. Eine derartige Ausgestaltung ermöglicht insbesondere diskrete Stufen der Längenveränderung. Bei einem ziehharmonikaartigen Faltteil sind vorzugsweise mehrere derartige Wellenstrukturen ausgebildet, die in Richtung der Längsachse auseinandergezogen oder zusammengeschoben werden können. Dadurch lässt sich diese Längenveränderung einstellen. Durch eine Ausgestaltung mit einem ziehharmonikaartigen Faltteil kann die Längenveränderungsvorrichtung auch einteilig ausgebildet sein. Dadurch kann Fertigungsaufwand und Montageaufwand eingespart werden. Insbesondere können in dem Zusammenhang auch Positionstoleranzen, die im Laufe der Zeit zwischen mehreren Teilelementen einer Längenveränderungsvorrichtung auftreten können, vermieden werden.

In einer vorteilhaften Ausführung ist vorgesehen, dass der Bügel zumindest bereichsweise als Schlauch ausgebildet ist. Eine derartige hohle Leitung ermöglicht einerseits eine Gewichtsreduzierung der Haptik. Andererseits wird durch diese Ausgestaltung auch der Bauraum geschaffen, um einerseits beispielsweise eine Teleskopverbindung realisieren zu können und/oder anderseits ein ziehharmonikaartiges Faltteil zu erzeugen und funktionell auch entsprechend betätigen zu können. Darüber hinaus ist durch eine derartige Ausgestaltung als hohler Schlauch auch die gewünschte Verformungselastizität des Bügels zumindest in Teilbereichen gegeben.

In einer vorteilhaften Ausführung ist vorgesehen, dass die Haptik ein zweites Haptikteil aufweist, welches zum ersten Haptikteil separat ausgebildet ist. Dieses zweite Haptikteil ist funktionell entsprechend dem ersten Haptikteil mit zumindest einer Längenveränderungsvorrichtung ausgebildet. Durch eine derartige Ausgestaltung lässt sich die Intraokularlinse verbessert in einem Kapselsack positionsstabil implantieren. Durch zumindest zwei separate Haptiken ist eine grundsätzliche Halterung der Intraokularlinse im Kapselsack erhöht. Indem dann auch noch beide Haptikteile mit zumindest jeweils einer Längenveränderungsvorrichtung ausgebildet sind, kann die bedarfsgerechte Einstellung der Größe der Intraokularlinse über die Verstellung der Längen von dem ersten Haptikteil und/oder dem zweiten Haptikteil besonders fein dosiert erfolgen. Das bedarfsgerechte Anpassen der Größen der Haptiken an die Gegebenheiten des Kapselsacks, in welchen die Intraokularlinse implantiert werden soll, ist dadurch sehr präzise ermöglicht. Dies sowohl im Hinblick auf die Länge als auch gegebenenfalls die Form.

Es kann vorgesehen sein, dass die beiden Haptikteile miteinander verbunden sind. In dem Zusammenhang kann die Stabilität der Haptik erhöht werden. Insbesondere ist vorgesehen, dass die beiden miteinander verbundenen Haptikteile einen umlaufenden Haptikring bilden. Dieser Haptikring ist somit vollständig geschlossen und daher unterbrechungsfrei ausgebildet. Bei einer derartigen Ausgestaltung bildet der Haptikring quasi einen Rahmen um den optischen Teil.

Insbesondere erstreckt sich eine Längsachse eines Haptikteils über die gesamte Länge betrachtet in einer Ebene. Insbesondere ist diese Ebene senkrecht zur optischen Hauptachse orientiert. Es kann vorzugsweise vorgesehen sein, dass sich die Längsachsen von zwei Haptikteilen, wenn zumindest eine derartige Anzahl von Haptikteilen ausgebildet ist, jeweils über ihre gesamte Länge in einer, insbesondere gemeinsamen, Ebene erstrecken.

In einer Ausführung kann vorgesehen sein, dass die Haptik direkt an dem optischen Teil angeordnet ist, insbesondere daran befestigt ist.

Es kann vorgesehen sein, dass die Intraokularlinse einstückig ausgebildet ist. Bei einer derartigen Ausgestaltung ist dann auch die Haptik mit dem optischen Teil einstückig ausgebildet, insbesondere aus einem Polymermaterial.

Es kann jedoch auch vorgesehen sein, dass die Intraokularlinse mehrteilig ausgebildet ist. Beispielsweise kann hier vorgesehen sein, dass der optische Teil eine erste Teilkomponente der Intraokularlinse darstellt und das zumindest eine Haptikteil eine dazu separate zweite Teilkomponente der Intraokularlinse darstellt. Diese beiden separaten Teilkomponenten können dann auf unterschiedliche Art und Weise verbunden sein.

Beispielsweise kann hier eine mechanische Verbindung, wie beispielsweise eine Steckverbindung oder eine Rastverbindung oder dergleichen, vorgesehen sein.

Insbesondere bei einer derartigen Ausgestaltung kann vorgesehen sein, dass der optische Teil am radial äußeren Randbereich einen Koppelsteg aufweist. Dieser Koppelsteg ist dann zum Eingreifen in diese Öffnung vorgesehen. Der Koppelsteg kann als Wulst oder Schiene ausgebildet sein. Insbesondere ist vorgesehen, dass dieser Koppelsteg ein zusätzlicher integrierter Bereich des optischen Teils ist, jedoch keine optische Abbildungseigenschaft aufweist. Er ist insbesondere lediglich zur mechanischen Kopplung mit der Haptik vorgesehen. Dadurch wird erreicht, dass die grundsätzliche Ausgestaltung des optischen Teils im Hinblick auf denjenigen Bereich, der die optische Abbildungseigenschaft liefert, nicht eingeschränkt wird beziehungsweise nicht beeinträchtigt ist. Insbesondere ist es dadurch dann auch vermieden, dass derjenige Bereich des optischen Teils, der für die optische Abbildungseigenschaft zuständig ist, nicht in die Öffnung eintaucht. Dadurch kann einerseits eine Reduzierung der optisch abbildenden Bereiche des optischen Teils vermieden werden und andererseits eine Beschädigung dieses optisch abbildenden Bereichs des optischen Teils oder ein Reiben oder dergleichen an der Öffnung des Bügels vermieden werden.

Es kann vorgesehen sein, dass ein derartiger Koppelsteg geradlinig ausgebildet ist. Er erstreckt sich dann nur um eine kleine Teillänge der gesamten Umlauflänge des äußeren radialen Rands des optischen Teils um die optische Hauptachse. Es kann jedoch auch vorgesehen sein, dass dieser Koppelsteg gekrümmt ist. Beispielsweise kann er einen radial äußeren Ring des optischen Teils darstellen, der zumindest bereichsweise umlaufend, insbesondere auch vollständig umlaufend, ausgebildet sein kann. Dadurch kann die azimutale Position des optischen Teils relativ zur Haptik individuell eingestellt werden. Dies ist insbesondere dann vorteilhaft, wenn der optische Teil beispielsweise auch zur Korrektur eines Astigmatismus ausgebildet ist. Denn dann kann eine individuelle Lage des optischen Teils relativ zur Haptik eingestellt werden, sodass einerseits die Haptik bestmöglich in dem Kapselsack positioniert werden kann, andererseits die Korrektur dieses Sehfehlers, insbesondere des Astigmatismus, für das individuelle Auge korrigiert werden kann.

In einer vorteilhaften Ausführung kann vorgesehen sein, dass die Längenveränderungsvorrichtung in diskreten Stufen in der Länge veränderbar ausgebildet ist. In dem Zusammenhang können die Längenveränderungen diskret beispielsweise in Millimeterschritten oder in Halbmillimeterschritten oder jedoch auch in Wertschritten, die größer als ein Millimeter sind, erfolgen. Es kann vorgesehen sein, dass zumindest zwei derartige diskrete Längenveränderungsstufen ausgebildet sind. Es können jedoch auch mehr als zwei, beispielsweise mehr als fünf oder beispielsweise mehr als zehn ausgebildet sein.

In einer alternativen Ausführung kann vorgesehen sein, dass eine Längenveränderungsvorrichtung in der Länge kontinuierlich verstellbar ist. Dadurch kann eine noch feinjustiertere Einstellung erfolgen.

Es kann vorgesehen sein, dass sich eine Längenveränderungsvorrichtung nur über eine Teillänge der gesamten Länge des Bügels bemisst. Beispielsweise kann diese Teillänge kleiner der Hälfte der Gesamtlänge des Bügels sein. Die Teillänge kann aber auch kleiner als ein Drittel, insbesondere kleiner als ein Viertel, der gesamten Länge eines Bügels sein.

Es kann vorgesehen sein, dass eine Längenveränderungsvorrichtung in einem Längenbereich des Bügels ausgebildet ist, der ein Endstück dieses Bügels darstellt. Die Längenveränderungsvorrichtung stellt bei einem derartigen Ausführungsbeispiel somit einen endseitigen Abschluss des Bügels entlang einer Längsachse betrachtet dar. Es kann vorgesehen sein, dass die Längenveränderungsvorrichtung örtlich an der Stelle im Bügel ausgebildet ist, mit welcher der Bügel mit dem optischen Teil mechanisch verbunden ist, insbesondere direkt verbunden ist. Eine Längenveränderungsvorrichtung kann somit in einem Längenabschnitt des Bügels ausgebildet sein, der in radialer Richtung zur optischen Hauptachse näher angeordnet ist als ein davon radial weiter entfernter Längenabschnitt. Insbesondere kann der radial näher zur optischen Hauptachse liegende Längenabschnitt des Bügels ein Endstück des Bügels sein.

Insbesondere wenn ein derartiger Bügel eine U-Form aufweist, kann eine Längenveränderungsvorrichtung das freie Ende eines U-Schenkels sein.

Insbesondere können bei einer derartigen Ausgestaltung die jeweiligen Enden der U-Schenkel als Längenveränderungsvorrichtung ausgebildet sein. Es kann in dem Zusammenhang vorgesehen sein, dass diese dann zumindest zwei Längenveränderungsvorrichtungen von der Bauart und somit von der Funktionalität gleich sind. Beispielsweise können in dem Zusammenhang zwei Teleskopverbindungen ausgebildet sein. Es können jedoch auch zwei ziehharmonikaartige Faltenteile ausgebildet sein.

Ebenso ist es möglich, dass als weitere konkrete Ausgestaltung einer Längenveränderungsvorrichtung nicht nur bei dieser Ausgestaltung Federelemente als Längenveränderungsvorrichtungen ausgebildet sind. Ein Federelement kann in dem Zusammenhang beispielsweise eine in Richtung der Längsachse elastisch verformbare Zylinderfeder sein. Diese kann beispielsweise aus einem Kunststoffmaterial ausgebildet sein. Ein Federelement kann auch mit einem weiteren Material umgeben sein. Beispielsweise kann es vollkommen umgeben sein. So kann in dem Zusammenhang auch vorgesehen sein, dass das Federelement von einem Polymermaterial umgeben ist. Es kann auch vorgesehen sein, dass ein derartiges Federelement von einem Material umspritzt ist.

Ein weiteres Ausführungsbeispiel für ein Federelement kann jedoch auch ein zylinderförmiger, massiver Formkörper sein, der in Richtung seiner Längsachse, die der Längsachse des Bügels entspricht, elastisch verformbar ist. Beispielsweise können hier entsprechend verformbare Kunststoffe genannt werden.

Bei einer derartigen Ausgestaltung kann ein derartiger zylinderförmiger Formkörper auch aus einem porösen Material ausgebildet sein. Dadurch kann das Gewicht reduziert werden und gegebenenfalls insbesondere die Verformungselastizität erhöht werden.

In einer alternativen Ausführung kann vorgesehen sein, dass dann, wenn die Haptik zumindest zwei separate Längenveränderungsvorrichtungen aufweist, diese aus unterschiedlicher Bauart und somit unterschiedlicher Funktionalität sind. Kombinationsmöglichkeiten sind in dem Zusammenhang beispielsweise einerseits durch eine Teleskopverbindung mit einem ziehharmonikaartigen Faltenteil oder einem Federelement möglich. Auch sind Kombinationen von einem ziehharmonikaartigen Faltteil und einem Federelement möglich.

Bei Ausführungen, bei welchen eine Längenveränderung in diskreten Längenstufen ermöglicht ist, kann die Längenveränderungsvorrichtung diesbezüglich Raststufen aufweisen. Dadurch können die eingestellten Längenstufen auch gehalten werden.

Es kann auch vorgesehen sein, dass die Längenveränderungsvorrichtung zwei Haptikteile aufweist, von denen jedes Haptikteil aus zwei separaten Teilelementen ausgebildet ist, die miteinander in Richtung der Längsachse längenveränderlich gekoppelt sind. Beispielsweise können hier für jedes Haptikteil zwei zumindest bereichsweise hohle Schläuche vorgesehen sein, die ineinander geführt sind und entsprechend mechanisch gekoppelt sind, um eine definierte Längenveränderung eines jeweiligen Haptikteils einstellen zu können. Es kann auch nur eines der beiden Teilelemente bereichsweise hohl sein, um das andere Teilelement einführen zu können.

Es kann auch vorgesehen sein, dass in dem fertigen Endzustand einer Intraokularlinse der optische Teil in Umlaufrichtung um die optische Hauptachse vollständig berührungslos zur Haptik angeordnet ist. Bei einer derartigen Ausgestaltung kann ein Verbindungselement, wie beispielsweise ein Drahtstück, zwischen dem optischen Teil und der Haptik, insbesondere dem strangartigen Bügel, ausgebildet sein. Dadurch wird eine gewisse Distanz zwischen dem optischen Teil und der Haptik erreicht, andererseits dennoch die stabile mechanische Verbindung zwischen der Haptik und dem dazu separaten optischen Teil ermöglicht.

Es kann auch vorgesehen sein, dass ein strangartiger Bügel, der ein Haptikteil darstellt, zumindest zwei separate und in Richtung der Längsachse beabstandet zueinander angeordnete Längenveränderungsvorrichtungen aufweist. Beispielsweise können diese beiden Längenveränderungsvorrichtungen jeweils beabstandet zu den Enden des insbesondere U-förmigen Bügels in dem Bügel selbst ausgebildet sein. Es kann jedoch auch vorgesehen sein, dass eine Längenveränderungsvorrichtung als Endstück eines derartigen Bügels ausgebildet ist und die zweite Längenveränderungsvorrichtung in Richtung der Längsachse des Bügels beabstandet dazu angeordnet ist. Beispielsweise kann hier in Bezug zur Gesamtlänge des Bügels diese weitere Längenveränderungsvorrichtung in etwa mittig in dem Bügel ausgebildet sein.

Ebenso kann ein symmetrischer Aufbau vorgesehen sein, bei welchem zwei Längenveränderungsvorrichtungen jeweils die Endstücke des insbesondere U-förmigen Bügels bilden und eine weitere dritte Längenveränderungsvorrichtung in Richtung der Längsachse des Bügels betrachtet beabstandet dazu, insbesondere in etwa mittig in Bezug zur Gesamtlänge des Bügels, in dem Bügel ausgebildet ist.

Es kann auch vorgesehen sein, dass in den Ausführungen, in denen in einem Bügel zumindest zwei separate Längenveränderungsvorrichtungen ausgebildet sind, diese Längenveränderungsvorrichtungen im Hinblick auf die Längeneinstellung unterschiedlich sind. Dies bedeutet, dass eine Längenveränderungsvorrichtung beispielsweise zur diskreten Veränderung der Länge in Längenstufen ausgebildet ist und die weitere Längenveränderungsvorrichtung zur kontinuierlichen Längenverstellung ausgebildet ist.

Es kann auch vorgesehen sein, dass ein Haptikteil zwei separate Teilelemente aufweist, die für sich betrachtet jeweils strangartig ausgebildet sind. Es kann vorgesehen sein, dass jedes Teilelement mit einem Endbereich mit dem optischen Teil, insbesondere an dessen Umfang, verbunden ist. Das jeweils andere, im nicht gekoppelten Zustand frei auskragende, Ende dieser Teileelemente ist dann insbesondere so gestaltet, dass sie ineinander geführt werden können. Dadurch kann eine entfernt von den Anbindungsstellen an dem optischen Teil ausgebildete Längenveränderungsvorrichtung, insbesondere eine Teleskopverbindung, einfach gestaltet werden. So kann in dem Zusammenhang die Intraokularlinse insbesondere einstückig, insbesondere aus einem Polymermaterial, gefertigt werden und im Nachgang diese frei auskragenden Enden der Teilelemente des Haptikteils ineinander geführt und somit verschiebbar gekoppelt werden. Dadurch ist der unterbrechungsfreie, insbesondere U-förmige, strangartige Bügel geschaffen, der eine integrierte Längenveränderungsvorrichtung, insbesondere als Teleskopverbindung, aufweist.

Auch diese Teleskopverbindung kann Raststufen aufweisen, um eine Teleskopverbindung nicht zur kontinuierlichen Verstellung, sondern zur diskreten Verstellung der Länge des Bügels auszubilden.

In einer vorteilhaften Ausführung ist vorgesehen, dass eine Längenveränderungsvorrichtung in den Bügel integriert ist. Insbesondere ist sie somit einstückig mit dem restlichen Teil des Bügels ausgebildet.

Insbesondere ist die Intraokularlinse als kapselsackimplantierende Intraokularlinse ausgebildet. Sie kann in dem Zusammenhang auch als Kapselsack-Implantations-Intraokularlinse bezeichnet werden. Insbesondere ist sie in dem Zusammenhang eine Hinterkammerlinse zum Implantieren in einen Kapselsack eines Auges. Dies bedeutet, dass die Intraokularlinse bestimmungsgemäß, insbesondere nur, zur Implantation in einen Kapselsack eines Auges vorgesehen ist.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

### Kurze Beschreibung der Zeichnungen

Ausführungsbeispiele der Erfindung werden nachfolgend anhand schematischer Zeichnungen näher erläutert. Es zeigen:
- Fig. 1: eine vereinfachte Darstellung eines Ausführungsbeispiels einer erfindungsgemäßen Intraokularlinse, die insbesondere in einem Kapselsack bereits implantiert ist;
- Fig. 2: eine vereinfachte Darstellung eines weiteren Ausführungsbeispiels einer erfindungsgemäßen Intraokularlinse, die insbesondere bereits in einem Kapselsack implantiert ist;
- Fig. 3: eine Darstellung gemäß Fig. 1 und Fig. 2 mit einem dritten Ausführungsbeispiel einer erfindungsgemäßen Intraokularlinse;
- Fig. 4: eine Darstellung gemäß Fig. 1 bis Fig. 3 mit einem vierten Ausführungsbeispiel einer erfindungsgemäßen Intraokularlinse;
- Fig. 5: eine Draufsicht sowie eine Seitenansicht eines optischen Teils einer Intraokularlinse mit spezifischen Koppelstegen zum Koppeln mit einer Haptik der Intraokularlinse;
- Fig. 6: eine Darstellung gemäß Fig. 5 mit dazu unterschiedlichen Ausführungen von Koppelstegen.
- Fig. 7: eine Draufsicht auf ein Ausführungsbeispiel einer Intraokularlinse, bei welcher die Haptikteile in einem Grundzustand mit ihren Längen gezeigt sind; und
- Fig. 8: eine Darstellung auf die Ausführung der Intraokularlinse gemäß Fig. 7, bei welcher die Haptikteile in der Länge vergrößert sind.

### Bevorzugte Ausführungsbeispiele der Erfindung

In den Figuren werden gleiche oder funktionsgleiche Elemente mit den gleichen Bezugszeichen versehen.

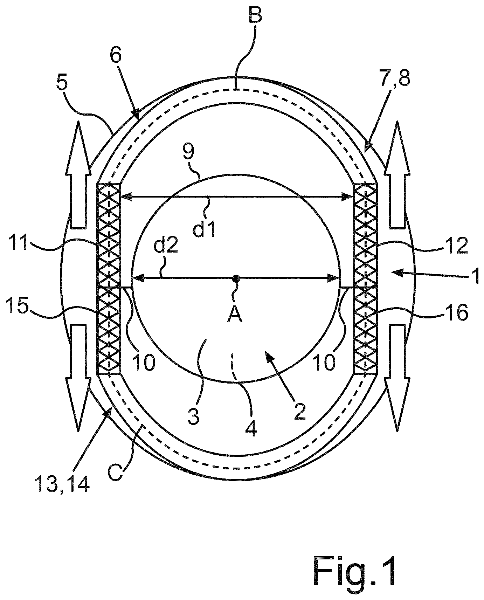

In Fig. 1 ist in einer perspektivischen Darstellung ein Ausführungsbeispiel einer künstlichen Intraokularlinse 1 gezeigt. Diese Intraokularlinse 1 ist eine Hinterkammerlinse zum Implantieren in einen Kapselsack eines Auges. Sie kann daher auch als Kapselsack-Implantations-Intraokularlinse bezeichnet werden. Die Intraokularlinse 1 weist einen optischen Teil 2 auf. Der optische Teil 2 ist als Linse ausgebildet. Er ist zur Erzeugung einer definierten optischen Abbildungseigenschaft der Intraokularlinse 1 ausgebildet. Die Intraokularlinse 1 weist eine optische Achse bzw. eine optische Hauptachse A auf. Diese durchdringt eine Vorderseite 3 des optischen Teils 2 und eine Rückseite 4 des optischen Teils 2 zentral und mittig des optischen Teils 2. Die optische Hauptachse A ist senkrecht zur Figurenebene orientiert.

Die Intraokularlinse 1, die in der Darstellung gemäß Fig. 1 beispielhaft in einem Kapselsack 5 implantiert gezeigt ist, ist darin positionsstabil gehalten. Dazu weist die Intraokularlinse 1 eine Haptik 6 auf, die spezifisch ausgebildet ist. Die Haptik 6 weist in dem Ausführungsbeispiel gemäß Fig. 1 ein erstes Haptikteil 7 auf. Dieses erste Haptikteil 7 ist als strangartiger Bügel 8 ausgebildet. Dieser Bügel 8 weist eine Längsachse B auf. Dieser Bügel 8 ist im Ausführungsbeispiel mit einer U-Form ausgebildet. Der Bügel 8 ist in einem Teilbereich bogenförmig ausgebildet, wobei dieser Teilbereich eine maximale Bogenweite d1 aufweist, die größer ist, als ein Durchmesser d2 des optischen Teils 2. Es kann vorgesehen sein, dass das erste Haptikteil 7 und somit der strangartige Bügel 8 direkt an dem optischen Teil 2, insbesondere an einem Umfangsrand 9, angeordnet ist. Es kann jedoch auch vorgesehen sein, wie dies bei der Darstellung in Fig. 1 vorgesehen ist, dass dieser Bügel 8 radial beabstandet zu dem optischen Teil 2 und somit auch zu dem Umfangsrand 9 angeordnet ist. Insbesondere dann, wenn der optische Teil 2 und die Haptik 6 separate Komponenten sind, kann eine derartige Ausgestaltung vorgesehen sein. Zum Verbinden der Haptik 6 mit dem optischen Teil 2 insbesondere einer derartigen beabstandeten Positionierung kann ein Verbindungselement 10 vorgesehen sein. Dieses Verbindungselement 10 kann beispielsweise ein Draht sein. Dazu kann vorgesehen sein, dass in dem Umfangsrand 9 eine Kerbe beziehungsweise eine Nut ausgebildet ist, in welche der Draht 10 eingelegt ist und dann seitlich radial abstehend nach außen geführt ist, um mit der Haptik 6 verbunden zu werden.

Der Bügel 8, der im Ausführungsbeispiel den optischen Teil 2 über eine Azimutlänge von insbesondere 180° umgibt, weist zumindest eine Längenveränderungsvorrichtung 11 auf. Im Ausführungsbeispiel weist der Bügel 8 zwei Längenveränderungsvorrichtungen 11 und 12 auf. Im Ausführungsbeispiel sind die Längenveränderungsvorrichtungen 11 und 12 als Endstücke dieses U-förmigen, strangartigen Bügels 8 ausgebildet. Die Längenveränderungsvorrichtungen 11 und 12 sind so ausgebildet, dass sie in Richtung der Längsachse B in ihrer Länge definiert veränderlich sind. Dadurch ist auch der Bügel 8 in seiner Länge in Richtung seiner Längsachse B betrachtet definiert veränderlich. Bei dem in Fig. 1 gezeigten Ausführungsbeispiel weist die Haptik 6 vorzugsweise ein weiteres, zweites Haptikteil 13 auf. Dieses ist in vorteilhafter Weise entsprechend zu dem ersten Haptikteil 7 ausgebildet. Es kann, wie auch das erste Haptikteil 7, zumindest bereichsweise hohl ausgebildet sein. Beispielsweise kann hier ein hohler Schlauch vorgesehen sein.

Im gezeigten Ausführungsbeispiel ist das zweite Haptikteil 13 ebenfalls als strangartiger Bügel 14 ausgebildet. Dieser strangartiger Bügel 14 weist eine Längsachse C auf. Insbesondere weist der strangartige Bügel 14 zumindest eine Längenveränderungsvorrichtung 15 auf. Insbesondere weist er bei dem dargestellten Ausführungsbeispiel ebenfalls zwei separate und auch hier beabstandet zueinander angeordnete Längenveränderungsvorrichtungen 15 und 16 auf. Diese sind in vorteilhafter Ausführung als Endstücke des U-förmigen Bügels 14 gestaltet.

Wie in der Darstellung von Fig. 1 zu erkennen ist, sind die beiden Bügel 8 und 14 in einer Ausführung an ihren jeweiligen distalen Enden direkt miteinander verbunden. Dadurch bilden sie einen umlaufend geschlossenen Bügel, der die Haptik 6 darstellt. Der optische Teil 2 ist somit durch einen umlaufend geschlossenen, strangartigen Gesamtbügel, der einen Haptikring darstellt, umgeben. Insbesondere erstrecken sich die Bügel 8 und 14 über ihre jeweils gesamte Länge entlang der Längsachse B, C betrachtet in einer Ebene, die insbesondere senkrecht zur optischen Hauptachse A orientiert ist.

Eine Längenveränderungsvorrichtung 11, 12, 15, 16 kann als Teleskopverbindung ausgebildet sein. Eine Längenveränderungsvorrichtung 11, 12, 15, 16 kann jedoch auch als ziehharmonikaartiges Faltteil, insbesondere vergleichbar einem Leporello, ausgebildet sein. Ebenso ist es möglich, dass eine Längenveränderungsvorrichtung 11, 12, 15, 16 als Federelement ausgebildet ist. In Fig. 1 ist vorgesehen, dass alle Längenveränderungsvorrichtungen 11, 12, 15, 16 gleicher Bauart und gleicher Funktionalität sind. Insbesondere sind die Längenveränderungsvorrichtungen 11, 12, 15, 16 als ziehharmonikaartige Faltteile ausgebildet. Durch deren Verstellbarkeit, die kontinuierlich oder diskret sein kann, kann die Länge eines Bügels 8 und/oder 14 in Richtung der jeweiligen Längsachse B und/oder C vergrößert oder verkleinert werden. Dies ist durch die symbolhaften Pfeile in Fig. 1 angedeutet.

Es kann auch vorgesehen sein, dass die Haptik 6 direkt mit dem optischen Teil 2, insbesondere dessen Umfangsrand 9, verbunden ist. Beispielsweise kann dazu vorgesehen sein, dass die Bügel 8 und/oder 14 auf der dem optischen Teil 2 zugewandten Seite Schlitze aufweisen. In diese Schlitze kann sich der optische Teil 2 hinein erstrecken und darin gehalten werden. Auch eine einstückige Ausgestaltung mit der Intraokularlinse 1 ist möglich. Bei dem in Fig. 1 gezeigten Ausführungsbeispiel sind die Längenveränderungsvorrichtungen 11, 12, 15 und 16 als Endstücke der jeweiligen Bügel 8, 14 ausgebildet. Es kann auch vorgesehen sein, dass zumindest eine Längenveränderungsvorrichtung 11, 12, 15, 16 nicht als ein derartiges Endstück ausgebildet ist, sondern beispielsweise mittig der Gesamtlänge eines Bügels 8, 14 darin ausgebildet ist. Wie zu erkennen ist, beträgt eine in Richtung der Längsachse B oder C betrachtete Länge einer Längenveränderungsvorrichtung 11, 12, 15, 16 maximal ein Drittel, insbesondere maximal ein Viertel, einer jeweiligen Gesamtlänge eines Bügels 8, 14.

Es kann auch vorgesehen sein, dass die Bügel 8 und 14 einstückig miteinander ausgebildet sind. Dadurch ist ein Bügelring gebildet, der vollständig umlaufend ausgebildet ist.

Insbesondere ist eine Längenveränderungsvorrichtung 11, 12, 15, 16 in einen Bügel 8, 14 integriert, insbesondere somit einstückig damit ausgebildet.

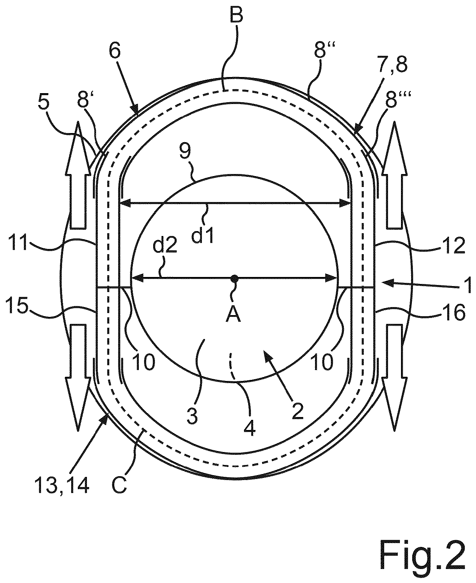

In Fig. 2 ist in einer entsprechenden Darstellung wie in Fig. 1 eine Intraokularlinse 1 gezeigt. Im Unterschied dazu sind die Längenveränderungsvorrichtungen 11, 12, 15, 16 nicht als ziehharmonikaartige Faltteile ausgebildet, sondern als Teleskopverbindungen. Das Haptikteil 7 mit dem strangartigen Bügel 8 ist hier aus zumindest zwei Teilelementen ausgebildet. Beispielsweise können auch drei Teilelemente vorgesehen sein. Dies ist ein erstes Teilelement 8', ein zweites Teilelement 8" und ein drittes Teilelement 8‴. Diese drei Teilelemente 8', 8", 8‴ sind zumindest bereichsweise ineinander geführt und können sich im ineinandergeführten Zustand in Richtung der Längsachse B relativ zueinander verschieben. Entsprechend kann der weitere strangartige Bügel 14 aufgebaut sein. Es kann auch vorgesehen sein, dass der Bügel 8 nur aus zwei relativ zueinander bewegbaren und ineinander geführten Teilelementen ausgebildet ist. Entsprechendes kann für den Bügel 14 vorgesehen sein. Bei einer derartigen Ausführung ist die gesamte Haptik 6 dann durch vier separate Teilelemente, insbesondere vier Schläuche, gebildet.

Die weiteren Erläuterungen zur Anordnung und Verbindung des optischen Teils 2 mit der Haptik 6 sind entsprechend Fig. 1 möglich. Auch bei der Ausgestaltung in Fig. 2 ist die Anzahl der Längenveränderungsvorrichtungen 11, 12, 15 und 16 lediglich beispielhaft zu verstehen, sodass auch hier mehr oder weniger als diese vier genannten vorgesehen sein können. Auch die jeweilige Position dieser Längenveränderungsvorrichtungen 11, 12, 15, 16 ist beispielhaft zu verstehen.

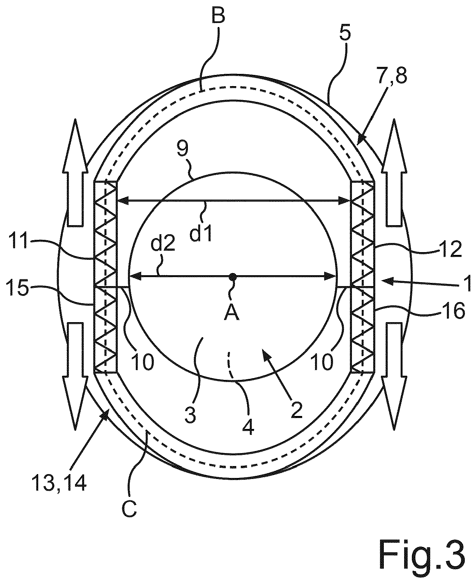

In Fig. 3 ist in einer Darstellung entsprechend Fig. 1 und Fig. 2 ein weiteres Ausführungsbeispiel einer Intraokularlinse 1 gezeigt. Im Unterschied zur Darstellung gemäß Fig. 1 und Fig. 2 sind hier, ebenfalls wiederum beispielhaft, vier Längenveränderungsvorrichtungen 11, 12, 15 und 16 vorgesehen, die hier Federelemente sind. Beispielsweise können hier axial federnde Zylinderfedern oder axial verformbare Hülsenelemente vorgesehen sein. Auch hier ist die Anzahl und Örtlichkeit der Längenveränderungsvorrichtungen 11, 12, 15, 16 beispielhaft zu verstehen. Auch hier gelten die alternativen Ausgestaltungsmöglichkeiten, wie sie zu Fig. 1 erläutert wurden, entsprechend.

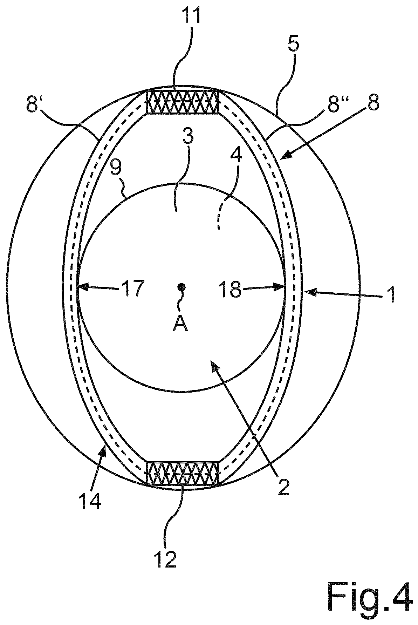

In Fig. 4 ist ein weiteres Ausführungsbeispiel einer Intraokularlinse 1 gezeigt. Auch diese ist beispielhaft bereits im implantierten Zustand in einem Kapselsack 5 dargestellt. Bei dieser schematischen Darstellung ist gezeigt, dass die Haptik 6 direkt mit dem optischen Teil 2, insbesondere am Umfangsrand 9, verbunden ist. Bei dieser Ausführung ist ein Beispiel gezeigt, bei welchem insbesondere eine einstückige Ausgestaltung der Intraokularlinse 1 vorgesehen sein kann. Insbesondere sind hier Längenveränderungsvorrichtungen 11 und 12 in den strangartigen Bügeln 8 und 14 entfernt von den Kopplungsstellen mit dem Umfangsrand 9 ausgebildet. Insbesondere sind diese Längenveränderungsvorrichtungen 11 und 12 auch nicht als Endstücke der U-förmigen Bügel 8 und 14 vorgesehen. Im Hinblick auf die U-Form der Bügel 8, 14 sind die Längenveränderungsvorrichtungen 11, 12 in etwa mittig der jeweiligen Gesamtlänge der Bügel 8 und 14 ausgebildet. Auch hier kann vorgesehen sein, dass die Bügel 8 und 14 einstückig ausgebildet sind und somit als umlaufend geschlossener einstückiger Haptikring ausgebildet sind.

Insbesondere ist hier vorgesehen, dass zumindest eine Längenveränderungsvorrichtung 11, 12 als Teleskopverbindung ausgebildet ist. Dieser Bügel 8 weist dazu zwei Teilelemente 8' und 8" auf, die an einem Ende mit dem optischen Teil 2 an den Verbindungsstellen 17 und 18, die insbesondere um 180° in Umlaufrichtung um die optische Hauptachse A versetzt zueinander ausgebildet sind, mit dem Umfangsrand 9 verbunden sind. Die anderen Enden dieser Teilelemente 8' und 8" sind diesbezüglich entfernt vom optischen Teil 2 ineinander geführt, sodass diesbezüglich eine Teleskopverbindung als Längenveränderungsvorrichtung 11 ausgebildet ist. Eine Längenveränderungsvorrichtung 11 und/oder 12 kann auch hier bei diesem Beispiel als ziehharmonikaartiges Faltteil oder als Federelement ausgebildet sein.

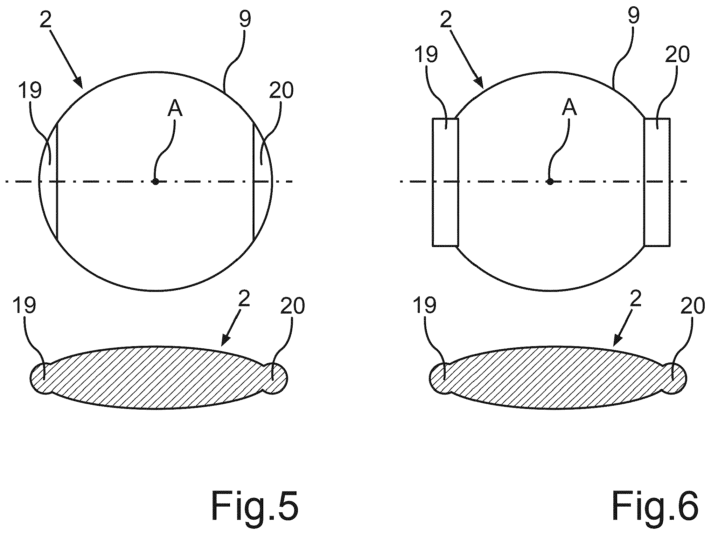

In Fig. 5 ist in einer vereinfachten Darstellung ein optischer Teil 2 in einer Draufsicht gezeigt. Es ist zu erkennen, dass an gegenüberliegenden Seiten Koppelstege 19 und 20 ausgebildet sind. Diese Koppelstege 19 und 20, die in der unteren Schnittdarstellung des optischen Teils 2 ebenfalls zu erkennen sind, dienen dazu, um in bereits oben angesprochene Öffnungen, insbesondere Schlitze, in den Bügeln 8 und/oder 14 aufgenommen zu werden. Dadurch kann der separate optische Teil 2 positionsstabil an der Haptik 6 befestigt werden. Die Schnittdarstellung in Fig. 5 ist lediglich beispielhaft zu verstehen. Die Schnittflächen der Koppelstege 19 und 20 sollen nur die jeweilige Geometrie verdeutlichen. Insbesondere ist der optische Teil 2 mit den Koppelstegen 19 und 20 einstückig ausgebildet. Die Koppelstege 19, 20 sind hier außenseitig so geformt, dass die gleichbleibende Kreisform des Umfangsrands 9 nicht verändert ist.

In Fig. 6 ist in einer entsprechenden Darstellung wie in Fig. 5 ein weiteres Ausführungsbeispiel eines optischen Teils 2 mit Koppelstegen 19 und 20 gezeigt. Im Unterschied zur Darstellung gemäß Fig. 5 ist bei der Ausführung in Fig. 6 ein in der Draufsicht geradliniger Koppelsteg 19 und 20 ausgebildet, der an seinen Enden nicht die Krümmung des Umfangrands 9 aufnimmt. In der unteren schematischen Schnittdarstellung in Fig. 6 ist wiederum eine Schnittdarstellung entlang der gestrichelten Linie der oberen Draufsicht in Fig. 6 gezeigt.

Bei dem hier gezeigten Beispiel für Koppelstege 19 und 20 sind diese teilweise umlaufend um die optische Hauptachse A ausgebildet. Es können auch Ausführungen vorgesehen sein, bei denen derartige Koppelstege als ein Koppelsteg ausgebildet ist und dieser vollständig umlaufend ausgebildet ist.

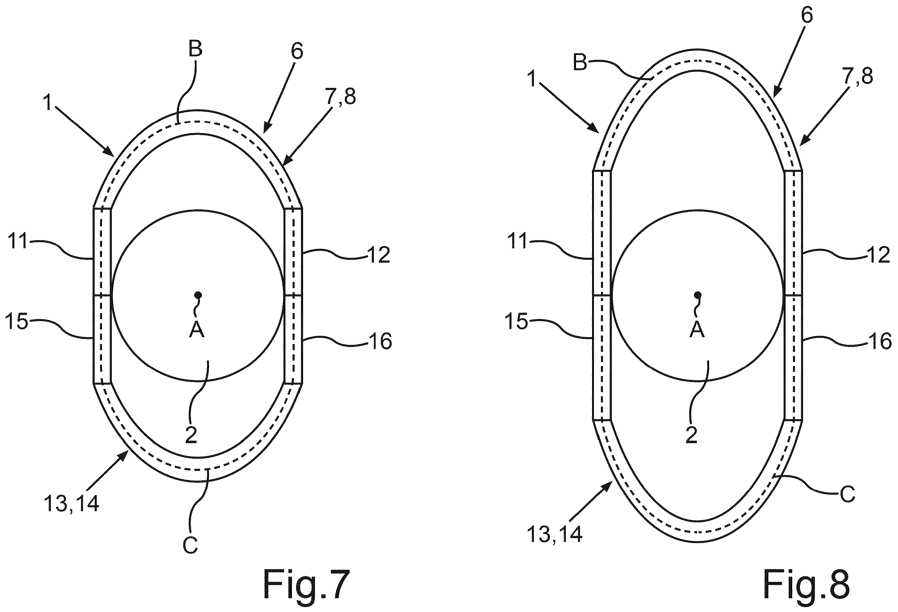

In Fig. 7 ist eine Draufsicht auf ein Ausführungsbeispiel einer Intraokularlinse 1 gezeigt. Die Haptikteile 7 und/oder 8 sind hier in einem Grundzustand bezüglich ihren Längen eingestellt. Das bedeutet, dass die Längenveränderungsvorrichtungen 11 und/oder 12 und/oder 15 und/oder 16 nicht vergrößert sind und somit nicht ausgezogen sind.

In Fig. 8 ist demgegenüber schematisch die Situation der Intraokularlinse gemäß Fig.7 gezeigt, bei welcher zumindest eine, insbesondere alle Längenveränderungsvorrichtungen 11, 12, 15, 16 in ihren Längen vergrößert sind. Es kann nicht nur in diesem Beispiel, sondern auch allgemein eine Längenvergrößerung vorgesehen sein, die bei allen Längenveränderungsvorrichtungen 11, 12 15, 16 gleich ist. Die Längenveränderungen der Längenveränderungsvorrichtungen 11, 12, 15, 16 können andererseits aber auch unterschiedlich sein.

Allgemein kann eine Längenveränderungsvorrichtung 11 und/oder 12 und/oder 15 und/oder 16 biegesteif sein oder auch, insbesondere elastisch, biegbar sein. So können dann insbesondere im verlängerten Zustand lange steife und gerade Teilstücke eines Haptikteils vermieden werden. Insbesondere kann durch ein Verbiegen auch eine Bogenform auch im Bereich einer Längenveränderungsvorrichtung fortgeführt werden.

## Patentansprüche

1. Intraokularlinse (1) mit einem optischen Teil (2) und mit einer Haptik (6), die mit dem optischen Teil (2) gekoppelt ist, und mit einer optischen Hauptachse (A), die eine Vorderseite (3) und eine Rückseite (4) des optischen Teils (2) durchdringt, wobei die Haptik (6) zumindest ein erstes Haptikteil (7) aufweist, welches als strangartiger Bügel (8) ausgebildet ist, wobei der strangartige Bügel (8) eine Längsachse (B) aufweist,
wobei der strangartige Bügel (8) zumindest eine Längenveränderungsvorrichtung (11, 12) aufweist, mit welcher der strangartige Bügel (8) in Richtung seiner Längsachse (B) in seiner Länge definiert veränderlich ist
**dadurch gekennzeichnet, dass**
der strangartige Bügel (8, 14) auf einer dem optischen Teil (2) zugewandten Seite eine Öffnung, insbesondere einen Schlitz, aufweist, in den der radial äußere Rand des optischen Teils (2) eingreift, so dass der optische Teil (2) in dem Haptikteil (7, 13) gehalten ist.

2. Intraokularlinse (1) nach Anspruch 1,
**dadurch gekennzeichnet, dass**
die Längenveränderungsvorrichtung (11, 12) zumindest eine Teleskopverbindung aufweist, welche sich in Richtung der Längsachse (B) elastisch in der Länge verändert.

3. Intraokularlinse (1) nach Anspruch 1 oder 2,
**dadurch gekennzeichnet, dass**
die Längenveränderungsvorrichtung (11, 12) zumindest ein zieharmonikaartiges Faltteil aufweist, welches sich in Richtung der Längsachse (B) elastisch in der Länge verändert.

4. Intraokularlinse (1) nach einem der vorhergehenden Ansprüche,
**dadurch gekennzeichnet, dass**
der strangartige Bügel (8) zumindest bereichsweise als Schlauch ausgebildet ist.

5. Intraokularlinse (1) nach einem der vorhergehenden Ansprüche,
**dadurch gekennzeichnet, dass**
die Haptik (6) ein zweites Haptikteil (13) aufweist, welches funktionell entsprechend dem ersten Haptikteil (7) mit einer Längenveränderungsvorrichtung (15, 16) ausgebildet ist.

6. Intraokularlinse (1) nach Anspruch 5,
**dadurch gekennzeichnet, dass**
die beiden Haptikteile (7, 13) miteinander verbunden sind und einen umlaufenden Haptikring bilden.

7. Intraokularlinse (1) nach einem der vorhergehenden Ansprüche,
**dadurch gekennzeichnet, dass**
die Längenveränderungsvorrichtung (11, 12, 15, 16) in diskreten Stufen in der Länge veränderbar ausgebildet ist.

8. Intraokularlinse (1) nach einem der vorhergehenden Ansprüche,
**dadurch gekennzeichnet, dass**
die Längenveränderungsvorrichtung (11, 12, 15, 16) in den strangartigen Bügel (8, 14) integriert ist

9. Intraokularlinse (1) nach einem der vorhergehenden Ansprüche,
**dadurch gekennzeichnet, dass**
die Intraokularlinse (1) eine Hinterkammerlinse zum Implantieren in einen Kapselsack (5) eines Auges ist.

## Claims

1. Intraocular lens (1) with an optical part (2) and with a haptic (6), which is coupled to the optical part (2), and with a main optical axis (A), which intersects a front side (3) and a back side (4) of the optical part (2), wherein the haptic (6) has at least one first haptic part (7), which is designed as a strand-like clip (8), wherein the strand-like clip (8) has a longitudinal axis (B),
wherein the strand-like clip (8) has at least one length-changing apparatus (11, 12) with which the strand-like clip (8) is variable in terms of its length in a defined manner in the direction of its longitudinal axis (B),
**characterized in that**
the strand-like clip (8, 14) has an opening, in particular a slot, on a side facing the optical part (2), into which opening the radially outer edge of the optical part (2) engages such that the optical part (2) is held in the haptic part (7, 13).

2. Intraocular lens (1) according to Claim 1,
**characterized in that**
the length-changing apparatus (11, 12) has at least one telescopic connection which changes elastically in length in the direction of the longitudinal axis (B).

3. Intraocular lens (1) according to Claim 1 or 2,
**characterized in that**
the length-changing apparatus (11, 12) has at least one accordion-like folding part which changes elastically in length in the direction of the longitudinal axis (B).

4. Intraocular lens (1) according to any one of the preceding claims,
**characterized in that**
the strand-like clip (8) is at least partially formed as a tube.

5. Intraocular lens (1) according to any one of the preceding claims,
**characterized in that**
the haptic (6) has a second haptic part (13), which is designed with a length-changing apparatus (15, 16) functionally corresponding to the first haptic part (7).

6. Intraocular lens (1) according to Claim 5,
**characterized in that**
the two haptic parts (7, 13) are connected to one another and form a circumferential haptic ring.

7. Intraocular lens (1) according to any one of the preceding claims,
**characterized in that**
the length-changing apparatus (11, 12, 15, 16) is designed to be variable in length in discrete steps.

8. Intraocular lens (1) according to any one of the preceding claims,
**characterized in that**
the length-changing apparatus (11, 12, 15, 16) is integrated into the strand-like clip (8, 14).

9. Intraocular lens (1) according to any one of the preceding claims,
**characterized in that**
the intraocular lens (1) is a posterior chamber lens for implantation into a capsular bag (5) of an eye.

## Revendications

1. Lentille intraoculaire (1) comportant une partie otique (2) et comportant une haptique (6) qui est accouplée à la partie optique (2), et présentant un axe principal optique (A) qui passe à travers une face avant (3) et une face arrière (4) de la partie optique (2), l'haptique (6) comportant au moins un premier élément haptique (7) qui est configuré sous forme d'arc (8) de type jonc, l'arc (8) de type jonc présentant un axe longitudinal (B),
l'arc (8) de type jonc comportant au moins un dispositif de modification (11, 12) de longueur, par lequel l'arc (8) de type jonc est modifiable, de manière définie, dans sa longueur en direction de son axe longitudinal (B)
**caractérisée en ce que**
l'arc (8, 14) de type jonc présente sur un côté faisant face à la partie optique (2) une ouverture, en particulier une fente, dans laquelle s'engage le bord radialement extérieur de la partie optique (2), de sorte que la partie optique (2) est maintenue dans l'élément haptique (7, 13).

2. Lentille intraoculaire (1) selon la revendication 1,
**caractérisée en ce que**
le dispositif de modification (11, 12) de longueur comporte au moins un raccord télescopique qui se modifie élastiquement en longueur en direction de l'axe longitudinal (B).

3. Lentille intraoculaire (1) selon la revendication 1 ou 2,
**caractérisée en ce que**
le dispositif de modification (11, 12) de longueur comporte au moins un élément pliable en accordéon, qui se modifie élastiquement en longueur en direction de l'axe longitudinal (B).

4. Lentille intraoculaire (1) selon l'une quelconque des revendications précédentes,
**caractérisée en ce que**
l'arc (8) de type jonc est au moins par zones configuré sous forme de tuyau.

5. Lentille intraoculaire (1) selon l'une quelconque des revendications précédentes,
**caractérisée en ce que**
l'haptique (6) comporte un second élément haptique (13) qui est fonctionnellement conforme au premier élément haptique (7) avec un dispositif de modification (15, 16) de longueur.

6. Lentille intraoculaire (1) selon la revendication 5,
**caractérisée en ce que**
les deux éléments haptiques (7, 13) sont raccordés l'un à l'autre et forment un anneau haptique continu.

7. Lentille intraoculaire (1) selon l'une quelconque des revendications précédentes,
**caractérisée en ce que**
le dispositif de modification (11, 12, 15, 16) de longueur est conçu pour être modifiable en longueur par paliers discrets.

8. Lentille intraoculaire (1) selon l'une quelconque des revendications précédentes,
**caractérisée en ce que**
le dispositif de modification (11, 12, 15, 16) de longueur est intégré dans l'arc (8, 14) de type jonc.

9. Lentille intraoculaire (1) selon l'une quelconque des revendications précédentes,
**caractérisée en ce que**
la lentille intraoculaire (1) est une lentille de chambre postérieure destinée à l'implantation dans un sac capsulaire (5) d'un œil.
